(19) Europäisches Patentamt
European Patent Office
Office européen des brevets

(11) Publication number: **0 477 871 A2**

(12) **EUROPEAN PATENT APPLICATION**

(21) Application number: **91116256.8**

(22) Date of filing: **24.09.91**

(51) Int. Cl.$^5$: **A61K 31/52**

(30) Priority: **28.09.90 JP 257004/90**
**28.09.90 JP 257005/90**

(43) Date of publication of application:
**01.04.92 Bulletin 92/14**

(84) Designated Contracting States:
**DE FR GB IT**

(71) Applicant: **NIPPON KAYAKU KABUSHIKI KAISHA**
**11-2, Fujimi 1-chome Chiyoda-ku**
**Tokyo 102(JP)**

(72) Inventor: **Shimada, Nobuyoshi**
**2-9-10, Shimo**
**Kita-ku, Tokyo(JP)**
Inventor: **Saijo, Masayuki**
**7, Minami, Nishirokujou**
**Nayoro-shi, Hokkaido(JP)**
Inventor: **Azuma, Masanobu**
**2-3, Midorigaoka**
**Asahikawa-shi, Hokkaido(JP)**

(74) Representative: **Türk, Gille, Hrabal**
**Brucknerstrasse 20**
**W-4000 Düsseldorf 13(DE)**

(54) **Antiviral composition, containing guanine derivatives.**

(57) An antiviral agent comprising at least two compounds selected from among:
(a) 9-(2-deoxy-2-hydroxymethyl-β-D-*erythro*oxetanosyl)guanine (oxetanocin G);
(b) 9-(2-hydroxyethoxymethyl)guanine (aciclovir); and
(c) (+)-9-[(1R,2R,3S)-2,3-bis(hydroxymethyl)cyclobutyl]guanine (carbocyclic oxetanocin G).

EP 0 477 871 A2

Background of the Invention

This invention relates to a novel antiviral composition having a selective and synergistic antiviral activity.

[Prior Art]

Herpes viruses 1 and 2 cause cephalitis and herpetic infection in, for example, cornea, labium oris and genitalia of adults and infants, particularly seriously in the case of infants.

Further, it has been recently found that the herpes virus group causes serious infective diseases.

In order to treat these diseases caused by herpes viruses, it is a general practice to use 9-(2-hydroxyethoxymethyl)guanine (hereinafter referred to as aciclovir or ACV). In recent years, it has been further proposed to use the aciclovir together with bromovinyldeoxyuridine (BVDU) (refer to Japanese Patent Laid-Open No. 120814/1985) or with ribonucleotide reductase inhibitor (refer to Japanese Patent Laid-Open No. 45423/1990).

However none of these treatments is satisfactory from the viewpoints of the efficacy and side effects. It has been therefore required to develop an antiviral agent having a better efficacy and a lower toxicity.

Summary of the Invention

Under these circumstances the present invention provides a novel antiviral composition having an excellent synergistic effect.

Accordingly the present invention provides an antiviral composition which comprises at least two compounds selected from the group consisting of:

(a) 9-(2-deoxy-2-hydroxymethyl-β-D-*erythro*oxetanosyl)guanine (hereinafter referred to as oxetanocin G or OXT-G) represented by the following formula (I):

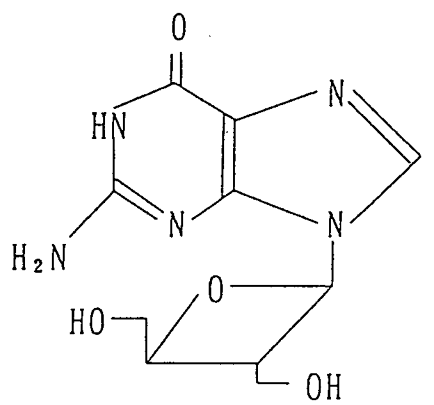

(I)

or a pharmacologically acceptable salt thereof;

(b) aciclovir represented by the following formula (II):

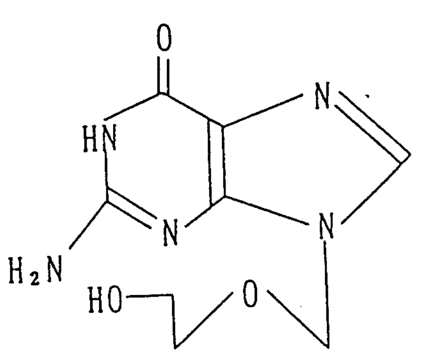

(II)

or a pharmacologically acceptable salt thereof; and

(c) (+)-9-[(1R,2R,3S)-2,3-bis(hydroxymethyl)cyclobutyl]guanine (hereinafter referred to as carbocyclic oxetanocin G or C-OXT-G) represented by the following formula (III):

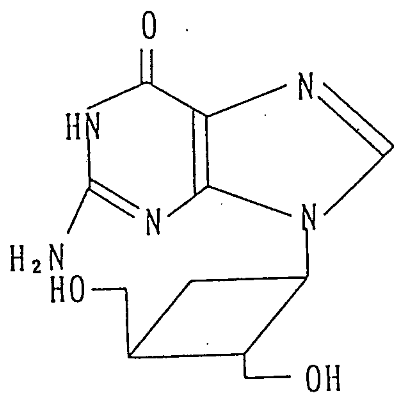

(III)

or a pharmacologically acceptable salt thereof.

Detailed Description of the Invention

The present inventors have found that an antiviral composition comprising at least two compounds selected from those represented by the aforesaid formulae (I), (II) and (III) is available as an anti-herpes virus composition since it exerts an excellent antiviral effect on various viruses, in particular, herpes viruses such as human herpes 1 or herpes 2, and a synergistic effect remarkably superior to the effect of use of each component alone.

The present invention has been accomplished based on the above finding. Each of the compounds to be used in the present invention is a known one. For example, oxetanocin G is disclosed in, for example, EP-A2-29197, aciclovir is now commercially available as an antiviral agent and described in, for example, Proc. Natl. Acad. Sci., 74, 5716 (1977), and carbocyclic oxetanocin G is disclosed in, for example, EP-A-358154.

In the present invention, the ratio of the component (a) (namely, oxetanocin G) to the component (b) (namely, aciclovir) or the component (c) (namely, carbocyclic oxetanocin G) may vary depending on the type of the herpes virus and the employed drug. This ratio, by weight, may range from 250 : 1 to 1 : 20, preferably from 150 : 1 to 1 : 10. It is still preferable to use 0.001 to 7 parts (by weight, the same will apply hereinafter), preferably 0.25 to 3 parts, of the component (b) or 0.005 to 4 parts, preferably 0.01 to 1 part, of the component (c) per 1 part of the oxetanocin G.

When the component (b) (namely, ACV) is used together with the component (c) (namely, C-OXT-G), the ratio of the component (c) to the component (b) may vary depending on the type of the virus. This ratio, by weight, may range from 20 : 1 to 1 : 200, preferably from 10 : 1 to 1 : 100. It is still preferable to use 0.02 to 6 parts of the component (c) per 1 part of the component (b).

Each of the oxetanocin G, aciclovir and carbocyclic oxetanocin G forms a salt with an acid. Any acid may be used for the formation of the salt, so long as it is pharmacologically acceptable. Preferable examples of the acid include hydrochloric, sulfuric and phosphoric acids. Alternately, a sodium or potassium adduct formed by using an alkaline solution such as sodium hydroxide or potassium hydroxide may be used in the present invention.

The antiviral composition of the present invention may be usually mixed with pharmaceutical additives such as fillers or carriers and administered in the form of, for example, injections, oral drugs or suppositories. The fillers and carriers are selected from among pharmaceutically acceptable ones, and the type and composition thereof depend on the administration route or administration method. Examples of liquid carriers include water, alcohols, animal and vegetable oils such as soybean oil, peanut oil, rubber oil and mineral oils and synthetic oils. Examples of solid carriers include sugars such as maltose and sucrose, amino acids, cellulose derivatives such as hydroxypropylcellulose and organic acid salts such as magnesium stearate. When the antiviral composition of the present invention is to be formulated into an injection, it is desirable to use physiological saline, various buffer solutions, solutions of sugars such as glucose, inositol or mannitol and glycols such as ethylene glycol and polyethylene glycol. It is also possible to lyophilize the antiviral composition of the present invention together with appropriate filler(s), for example, sugars such as inositol, mannitol, glucose, mannose, maltose or sucrose or amino acids such as phenylalanine. Then the lyophilized composition may be dissolved in a solvent suitable for intravenous injection, for example, sterilized water, physiological saline, a glucose solution, an electrolytic solution or an amino acid prior to the administration.

The total content of the component (a) (oxetanocin G) and the component (b) (aciclovir) or the component (c) (carbocyclic oxetanocin G) may widely vary depending on the formulation. In general, this content may range from 0.1 to 100% by weight, preferably from 1 to 90% by weight. For example, an injection may usually contain 0.1 to 5% by weight of the each of the components (a) and (b) or (c). When the antiviral composition of the present invention is to be orally administered, it may be formulated into, for example, tablets, capsules, dusts, granules, solutions or dry syrups together with the pharmaceutical additives such as the aforesaid liquid or solid carrier(s). Generally speaking, the capsules, tablets, granules and dusts comprise approximately 3 to 100% by weight, preferably 5 to 90% by weight, of the components (a) and (b) and the balance of the pharmaceutical additives such as the carrier(s).

The dose is determined depending on, for example, the age, body weight and conditions of the patient and the object of the treatment. The antiviral composition of the present invention may be generally administered in a dose of from 1 to 50 mg/kg per day [on the basis of the total amount of the components (a) and (b) or (c); the same will apply hereinafter] in the case of parenteral administration and from 5 to 50 mg/kg per day in the case of oral administration.

The oxetanocin G, aciclovir and carbocyclic oxetanocin G are lowly toxic and each of these compounds in further characterized by a low cumulative toxicity even in repetitive administration. When 400 mg/kg of oxetanocin G is intraperitoneally administered to a mouse at once, for example, no sign of toxicity is observed.

Now Formulation Examples of the present invention will be given.

Formulation Example 1

Purified water was added to 250 parts by weight of each of oxetanocin G and aciclovir or carbocyclic oxetanocin G and the pH value of the mixture was adjusted to 13 to 14 and the total amount thereof was adjusted to 2000 parts. After dissolving the components, the obtained solution was filtered by using a Millipore filter of GS type for sterilization. 2 g of the filtrate was introduced into a 10-ml vial and lyophilized. Thus a lyophilized injection containing 50 mg/vial of each of oxetanocin G and aciclovir or carbocyclic oxetanocin G was obtained.

Formulation Example 2

Granules:

50 parts by weight of each of oxetanocin G and aciclovir or carbocyclic oxetanocin G, 600 parts of lactose, 330 parts of crystalline cellulose and 20 parts of hydroxypropylcellulose were well mixed together, compressed with a roll-type compressor (Roller Compactor, trade name) and ground. Next, the particles were sieved to collect those particles which pass through a 16-mesh sieve but not through a 60-mesh sieve to thereby give granules.

Formulation Example 3

Tablets:

50 parts by weight of each of oxetanocin G and aciclovir or carbocyclic oxetanocin G, 120 parts of crystalline lactose, 147 parts of crystalline cellulose and 3 parts of magnesium stearate were tableted in a V-type mixer to thereby give tablets each weighing 300 mg and containing approximately 50 mg of the active ingredients.

Formulation Example 4

Purified water was added to 250 parts by weight of each of carbocyclic oxetanocin G and aciclovir and the pH value of the mixture was adjusted to 13 to 14 and the total amount thereof was adjusted to 2000 parts. After dissolving the components, the obtained solution was filtered by using a Millipore filter of GS type for sterilization. 2 g of the filtrate was introduced into a 10-ml vial and lyophilized. Thus a lyophilized injection containing 50 mg/vial of each of carbocyclic oxetanocin G and aciclovir was obtained.

Formulation Example 5

Granules:

50 parts by weight of each of carbocyclic oxetanocin G and aciclovir, 600 parts of lactose, 330 parts of crystalline cellulose and 20 parts of hydroxypropylcellulose were well mixed together, compressed with a roll-type compressor (Roller Compactor, trade name), and ground. Next, the particles were sieved to collect those particles which pass through a 16-mesh sieve but not through a 60-mesh sieve to thereby give granules.

Test Example

To further illustrate the Synergistic effects of the compositions of the present invention in inhibiting the growth of herpes simplex viruses 1 and 2, the following Test Example will be given.

(1) Method for determining the growth inhibition effect:

A monolayer culture of Cercopithecus sabaeus renal cells (vero cells) was prepared in Eagle's minimum medium containing 10% of newborn calf serum (MEM-CS10) by using a 24-well microplate. Then the medium was inoculated with 50 PFU/0.1 ml/well of herpes simplex virus 1 (HSV-1) or 2 (HSV-2). After effecting adsorption at 37° C for 1 hour, the cells in each well were washed with a phosphate buffer solution (PBS). Next, 0.5% methylcellulose-containing MEM-CS2 containing stepwise diluted OXT-G, ACV or C-OXT-G alone, a combination of OXT-G and ACV, a combination of OXT-G and C-OXT-G or a combination of ACV and C-OXT-G was laminated thereon and incubated at 37° C for 2 days. After discarding the laminated medium and fixing the cells with formation, the cells were stained with a Crystal Purple solution and plaques formed in each well were counted. Then the ratio (%) of the plaque count at each concentration to that of the control lot, to which no chemical was added, was determined to thereby give the 50% plaque inhibition concentration ($IC_{50}$). Thus the inhibitory rate (%) against the concentration of each compound was calculated.

(2) Results

Table 1: Synergistic effect of the combined use of OXT-G and ACV on HSV-1 (VR-3)

| Concentraten (μg/mℓ) of combined use at 50% plaque inhibition and inhibitory rate of each single compound at the same concn. | | | | Actual inhibitory rate of combined use | Theoretical inhibitory rate of combined use |
|---|---|---|---|---|---|
| OXT-G concn. | OXT-G inhibitory rate | ACV concn. | ACV inhibitory rate | | |
| 0 | 0 | 1.4 | 50% | - | - |
| 0.4 | (3%) | 0.42 | (15%) | 50% | (18%) |
| 1.0 | (7%) | 0.32 | (12%) | 50% | (19%) |
| 4.0 | (29%) | 0.08 | (3%) | 50% | (32%) |
| 5.4 | (39%) | 0.04 | (2%) | 50% | (41%) |
| 7.0 | 50% | 0 | 0 | - | - |
| OXT-G concn. | OXT-G inhibitory rate | C-OXT-G concn. | C-OXT-G inhibitory rate | Actual inhibitory rate of combined use | Theoretical inhibitory rate of combined use |
| 0 | 0 | 0.60 | 50% | - | - |
| 0.1 | (1%) | 0.4 | (33.3%) | 50% | (34%) |
| 1.7 | (12%) | 0.1 | (8%) | 50% | (20%) |
| 3.0 | (26%) | 0.04 | (3%) | 50% | (29%) |
| 7.3 | 50% | 0 | - | - | - |
| C-OXT-G concn. | C-OXT-G inhibitory rate | ACV concn. | ACV inhibitin rate | Inhibitory rate of combined use | Theoretical inhibitory rate of combined use |
| 0 | 0 | 0.78 | 50% | - | - |
| 0.01 | (2%) | 0.58 | (37%) | 50% | (39%) |
| 0.2 | (33%) | 0.1 | (6%) | 50% | (39%) |
| 0.3 | 50% | 0 | 0 | - | - |

*Note: The figures in the parentheses refer to the theoretical inhibitory rate.

Table 2: Synergistic effect of the combined use of OXT-G and ACV on HSV-2 (VW-268)

| Concentraten (µg/mℓ) of combined use at 50% plaque inhibition and inhibitory rate of each single compound at the same concn. | | | | Actual inhibitory rate of combined use | Theoretical inhibitory rate of combined use |
|---|---|---|---|---|---|
| OXT-G concn. | OXT-G inhibitory rate | ACV concn. | ACV inhibitory rate | | |
| 0 | 0 | 3.4 | 50% | - | - |
| 0.4 | (2%) | 2.7 | (40%) | 50% | (42%) |
| 1.0 | (5%) | 1.9 | (28%) | 50% | (33%) |
| 4.0 | (20%) | 1.3 | (19%) | 50% | (39%) |
| 10.0 | (50%) | 0 | 0 | - | - |
| C-OXT-G concn. | C-OXT-G inhibitory rate | ACV concn. | ACV inhibitory rate | Actual inhibitory rate of combined use | Theoretical inhibitory rate of combined use |
| 0 | 0 | 2.5 | 50% | - | - |
| 0.04 | (3%) | 1.8 | (36%) | 50% | (39%) |
| 0.1 | (7%) | 1.5 | (30%) | 50% | (37%) |
| 0.3 | (20%) | 0.4 | (8%) | 50% | (28%) |
| 0.54 | (36%) | 0.1 | (2%) | 50% | (38%) |
| 0.74 | 50% | 0 | 0 | - | - |

Effect:

As the above results clearly show, the combined use of these two components attained a 50% inhibitory rate at a concentration of each compound giving a theoretical (calculated) inhibitory rate of 18 to 42%. Thus it has been proved that the combined use exerts a remarkable synergistic effect.

## Claims

**1.** An antiviral composition comprising at least two compounds selected from the group consisting of:
(a) 9-(2-deoxy-2-hydroxymethyl-β-D-erythrooxetanocyl) guanine (oxetanocin G) represented by the following formula (I):

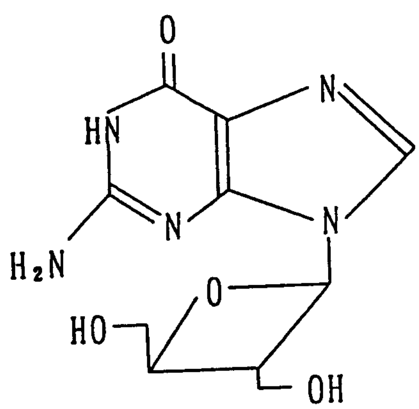

(I)

or a pharmacologically acceptable salt thereof;
(b) 9-(2-hydroxyethoxymethyl)guanine (aciclovir) represented by the following formula (II):

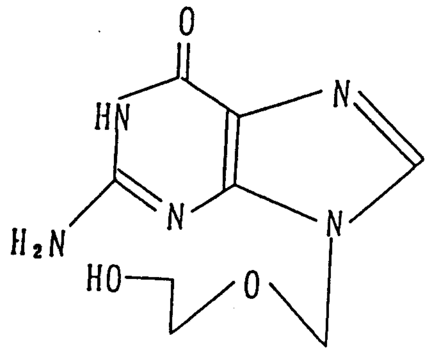

(II)

or a pharmacologically acceptable salt thereof; and

(c) (+)-9-[(1R,2R,3S)-2,3-bis(hydroxymethyl)cyclobutyl]guanine (carbocyclic oxetanocin G) represented by the following formula (III):

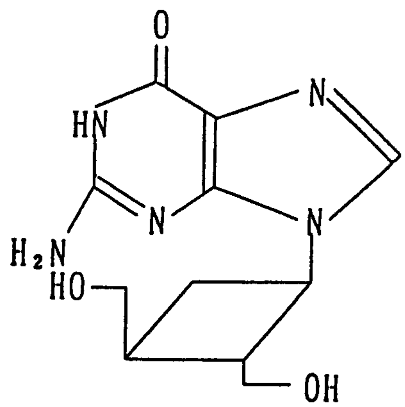

(III)

or a pharmacologically acceptable salt thereof.

2. A composition as claimed in Claim 1, which comprises oxetanocin G of the formula (1) and aciclovir of the formula (II).

3. A composition as claimed in Claim 2, which comprises from 0.01 to 7 parts by weight of aciclovir of the formula (II) per part by weight of oxetanocin G of the formula (I).

4. A composition as claimed in Claim 3, wherein the ratio by weight of oxetanocin G of the formula (I) to aciclovir of the formula (II) is 1 : 0.25 to 3.

5. A composition as claimed in Claim 1, which comprises oxetanocin G of the formula (I) and carbocyclic oxetanocin G of the formula (III).

6. A composition as claimed in Claim 5, which comprises from 0.005 to 4 parts by weight of carbocyclic oxetanocin G of the formula (III) per part by weight of oxetanocin G of the formula (I).

7. A composition as claimed in Claim 6, wherein the ratio by weight of oxetanocin G of the formula (I) to carbocyclic oxetanocin G of the formula (III) is 1 : 0.001 to 1.

8. A composition as claimed in Claim 1, which comprises aciclovir of the formula (II) and carbocyclic oxetanocin G of the formula (III).

9. A composition as claimed in Claim 8, wherein the ratio by weight of aciclovir of the formula (II) to carbocyclic oxetanocin G of the formula (III) is 1 : 0.02 to 6.

**10.** Use of compositions of claim 1 in treating or preventing diseases caused by virus, particularly herpes simplex virus.